# EUROPEAN PATENT SPECIFICATION

(11) **EP 1 489 170 B1**
(45) Date of publication and mention of the grant of the patent: **11.08.2010**
(21) Application number: 03708580.0
(22) Date of filing: 13.03.2003
(51) Int. Cl.: C12N 15/09, C07K 14/47, C12N 5/10, C12N 1/19, C12N 1/21, C07K 16/18, C12Q 1/02, C12Q 1/68, A61K 38/00, A61K 39/395, A61P 39/02, A61P 43/00

(54) **TRANSPORTER SELECTIVELY TRANSPORTING SULFATE CONJUGATE AND ITS GENE**
SULFATKONJUGAT SELEKTIV TRANSPORTIERENDER TRANSPORTER UND SEIN GEN
TRANSPORTEUR TRANSPORTANT SELECTIVEMENT UN CONJUGUE DE SULFATE ET SON GENE

(30) Priority: 14.03.2002 JP 2002070985
(43) Date of publication of application: 22.12.2004
(73) Proprietor: J-Pharma Co., Ltd., Tokyo 160-0022 (JP)
(72) Inventor: ENDOU, Hitoshi, Sagamihara-shi, Kanagawa 229-0022 (JP); KANAI, Yoshikatsu, Hachioji-shi, Tokyo 193-0931 (JP)
(74) Representative: Benson, John Everett
(86) International application number: PCT/JP2003/002980
(87) International publication number: WO 2003/076617

(56) References cited:
- WO-A-00/61755
- WO-A1-00/75315
- WO-A2-01/04283
- WO-A2-01/46258
- JP-A- 2002 171 980
- DATABASE EBI [Online] 28 August 1998 (1998-08-28), XP002331730 retrieved from EBI Database accession no. AI040384
- DATABASE UniProt [Online] 1 March 2003 (2003-03-01), XP002331729 retrieved from EBI Database accession no. Q8IVM8
- DATABASE UniProt [Online] 1 March 2002 (2002-03-01), XP002331731 retrieved from EBI Database accession no. Q8WYN7
- ABE T. ET AL.: 'Identification of a novel gene family encoding human liver-specific organic anion transporter LST-1' J. BIOL. CHEM. vol. 274, no. 24, 1999, pages 17159 - 17163, XP002191048
- KEPPLER D. ET AL.: 'Hepatic canalicular membrane 5: expression and localization of the conjugate export pump encoded by the MRP2 (cMRP/cMOAT) gene in liver' FASEB J. vol. 11, no. 7, 1997, pages 509 - 516, XP002969947
- CHA SH. ET AL.: 'Molecular cloning and characterization of multispecific organic anion transporter 4 expressed in the placenta' J. BIOL. CHEM. vol. 275, no. 6, 2000, pages 4507 - 4512, XP002188135

## Description

The present invention relates to a protein participating in a transport of a sulfate conjugate in the liver and a gene encoding the protein. Also, the invention relates to a method of modifying pharmacokinetics by altering an ability of the protein of selectively transporting the sulfate conjugate with the use of the protein participating in the transport of a sulfate conjugate in the liver or its specific antibody.

The liver plays an important role in metabolism and excretion of xenobiotics and drugs. The hepatic cell is the epithelial cell having a polarity, which contacts blood via the basolateral (sinusoidal) plasma membrane to deliver various substances. A drug in the portal vein blood is taken into the hepatic cell via a transporter or via the basolateral plasma membrane by way of passive diffusion through the cell membrane lipid bilayer and then subjected to a conjugation reaction in the cell owing to the metabolism to be a conjugate.

Examples of the conjugation reaction are a glutathione conjugation, a glucuronide conjugation, a sulfate conjugation, and the like, and a generated conjugate becomes an anion (negative ion) which is recognized by an organic anion transporter existing in the hepatic cell membrane to be excreted from the hepatic cell. The glutathione conjugate and the glucuronide conjugate are excreted via the luminal membrane (near the cholangiole) of the hepatic cell to the cholangiole. The sulfate conjugate is excreted mainly from the basolateral plasma membrane of the hepatic cell into blood.

Xenobiotics other than drugs and steroid hormones are metabolized by a mechanism similar to that of the drug metabolism in the hepatic cells to be excreted as conjugates.

The sulfate conjugates of the drugs, xenobiotics, and steroid hormones excreted into blood are taken up by the organic anion transporters existing in the basolateral plasma membranes of the proximal tubules epithelial cells in the kidney and then excreted into urine by the organic anion transporters in the luminal membranes of the tubules epithelial cells.

As one of the organic anion transporters in the luminal membrane of the hepatic cell, the canalicular multiselective organic anion transporter (cMOAT) has already been cloned.

The organic anion uptake via the basolateral plasma membrane of the hepatic cell has been studied using an experiment system employing removed organ perfusion or isolated cell membrane patches.

However, with the conventional methods, it is difficult to analyze in detail the organic anion transport via cell membrane, particularly a transport mechanism responsible for the excretion of the organic anion produced by the metabolism in the cell, and there has been a demand for a detailed analysis of an isolated transporter.

In view of the above-described background, exploration of molecule entity of the organic anion transporter in the basolateral plasma membrane of the liver has been actively conducted in the 1990s. As a result of the exploration, organic anion transporter oatp in the basolateral plasma membrane of the liver was isolated (Hagenbuch, B. et al., Proc. Natl. Acad. Sci. USA, Vol. 88, pp. 10629-33, 1991; Jacquemin, E. et al., Proc. Natl. Acd. Sci. USA, Vol. 91, pp. 133-7, 1994). However, the transporter was responsible for a transport of bile acids and the like and was different from that transporting drug conjugates.

Further, in 1997, organic anion transporter OAT1 responsible for an organic anion transport in the kidney was isolated (Sekine, T. et al., J. Biol. Chem. Vol. 272, pp. 18526-9, 1997). OAT1 transports a part of drug conjugates, but expression thereof in the liver has not been observed.

Following OAT1, organic anion transporter OAT2 which has a structure similar to that of OAT1 and is expressed in the liver has been isolated (Sekine, T. et al., Biochem. Biophys. Res. Commun. 251: 586-591, 1998). However, OAT2 has a low transport activity against the conjugate drugs.

Further, organic anion transporter OAT3 which has a structure similar to that of OAT1 and is expressed in the kidney, the liver, and the brain has been conceived (Kusuhara, H. et al. J. Biol. Chem., 274: 13675-13680, 1999). OAT3 is a multiselective transporter including drug conjugates, having substrates of various organic anions and exhibiting a remarkably wide range of substrate selectivity. For the purposes of efficient drug conjugate excretion via the basolateral plasma membrane of the hepatic cell, it is desirable that an organic anion transporter dedicated for drug conjugates is present in addition to OAT3 to avoid competition with other organic anions.

Following OAT3, organic anion transporter OAT4 which has a structure similar to that of OAT1 and is expressed in the kidney and the placenta was isolated (Cha, S. H. et al., J. Biol. Chem. 275: 4507-4512 (2000)). OAT4 exhibits a relatively sulfate conjugate-selective substrate selectivity mainly toward sulfate conjugates and including a part of organic anions, but OAT4 is not expressed in the liver.

As is previously stated, the organic anion transport in the basolateral plasma membrane in the liver is complicated, and particularly an excretion pathway through which conjugates (many of them are organic anions) produced in large quantity in the hepatic cells are excreted into blood is still unknown. The excretion pathway cannot be fully explained only by the organic anion transporters expressed in the liver, and an existence of an unknown transporter is expected.

WO 01/04283 discloses a novel human organic transporter polypeptide which can be used for pharmacological screening of substrates, analogues of substrates and modulators.

An object of the present invention is to identify and provide a novel organic anion transporter gene which selectively transports a sulfate conjugate in the hepatic cell basolateral plasma membrane and an organic anion transporter which is a polypeptide encoded by the gene. Other objects will become apparent from the following description.

The present inventors have searched the EST (expressed sequence tag) database using a base sequence of a translation region of a cDNA of OAT1 to identify a base sequence similar to that of OAT1. A probe equivalent to the identified base sequence was produced to conduct a screening of a cDNA library, and a gene encoding a novel protein was cloned. Further, by expressing a product of the gene in an oocyte of *Xenopus laevis*, it has been proved that the gene product is the novel organic anion transporter which selectively transports a sulfate conjugate. Furthermore, an antibody specific to the gene product was prepared to clarify that the gene product exists in the hepatic cell basolateral plasma membrane, thereby the present invention was accomplished.

The invention provides a protein comprising the amino acid sequence represented by SEQ ID NO: 1 and being capable of selectively transporting a sulfate conjugate

The invention also provides a gene encoding a protein of the invention or a gene comprising a DNA comprising the base sequence represented by SEQ ID NO: 2 and a DNA encoding a protein capable of selectively transporting a sulfate conjugate.

The live-specific organic anion transporter OAT7 which selectively transports a sulfate conjugate is expressed only in the liver in the living body and present in the basolateral plasma membrane of the hepatic cell. OAT7 is considered to be the transporter which excretes into blood sulfate conjugates of drugs, xenobiotics, and steroid hormones, and the like, generated by intracellular metabolism in the hepatic cells.

### Brief description of the Drawings

Fig. 1 shows a comparison of the amino acid sequences of human OAT7, human OAT1, rat OAT2, human OAT3, and human OAT4. An expected membrane-spanning section is indicated by a line. An expected glycosylation site and an expected C-kinase-dependent phosphorylation site are indicated by * and #, respectively.
Fig. 2 shows a phylogenetic tree based on amino acid sequences of human OAT7, human OAT1, rat OAT2, human OAT2, human OAT3, human OAT4, and amino acid sequences of human OCT1, human OCT2, and human OCT3, which are organic cation transporters having a structure similar to that of OAT.
Fig. 3 shows an exon-intron structure of a human OAT 7 chromosome gene.
Fig. 4 is a photograph substituting for a drawing showing a result of an analysis of expression of an OAT7 gene mRNA in the human organ and tissues by northern blotting. A: the adult tissue and placenta, B: the fetal tissue.

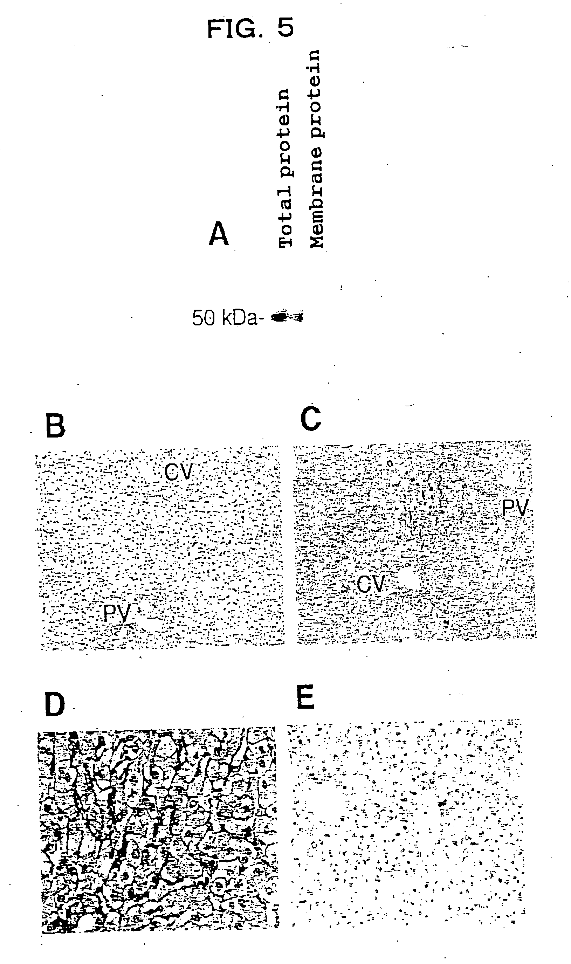

Fig. 5A is a photograph substituting for a drawing showing a result of a western blotting analysis of a human liver protein by an anti-OAT7 antibody. Figs. 5B to 5E are photographs substituting for drawings showing a result of an immunohistochemical analysis of OAT7 in the human liver by the anti-OAT7 antibody. B: a reaction without the anit-OAT7 antibody, in which no staining is observed; C: a slightly magnified image of staining by the anti-OAT7 antibody, in which the staining is observed all over the liver; D: a highly magnified image of the staining by the anti-OAT7 antibody, in which the staining is observed in the basolateral plasma membrane of hepatic cells; E: an absorption experiment using an antigen peptide, in which the staining detected in Fig. 5D has disappeared to exhibit the specificity of the staining.
Fig. 6A show results of experiments of [³H]estrone-sulfate conjugate uptake and [³H]dehydroepiandrosterone-sulfate conjugate uptake by an oocyte into which the human OAT7 gene cRNA was injected. Fig. 6B shows a change with time of the [³H]estrone-sulfate conjugate uptake by an oocyte into which the human OAT7 gene cRNA was injected. Fig. 6C shows a result of examination of the effect of salt added in the experiment of [³H]estrone-sulfate conjugate uptake by an oocyte into which the human OAT7 gene cRNA was injected.
Fig. 7 shows a result of examination of the effect of concentrations of the substrate estrone-sulfate conjugate and the dehydroepiandrosterone-sulfate conjugate in the experiments of uptakes of the [³H]estrone-sulfate conjugate and the [³H]dehydroepiandrosterone-sulfate conjugate by an oocyte into which the human OAT7 gene cRNA was injected.
Fig. 8 shows a result of examination of the effect of addition of various organic anions and organic cations to the system in the experiment of uptake of the [³H]estrone-sulfate conjugate by an oocyte into which the human OAT7 gene cRNA was injected.
Fig. 9 shows a result of examination of the effect of addition of organic anions considered to be transported in various hepatic cells to the system in the experiment of uptake of the [³H]estrone-sulfate conjugate by an oocyte into which the human OAT7 gene cRNA was injected.
Fig. 10 shows a result of examination of the effect of addition of various conjugate compounds to the system in the experiment of uptake of the [³H]estrone-sulfate conjugate by an oocyte into which the human OAT7 gene cRNA was injected.

### Detailed Description

A total length of a cDNA base sequence (about 2.3 kbp) of a gene of a liver-specific organic anion transporter (human OAT7) which selectively transports a human liver-derived sulfate conjugate and an amino acid sequence (533 amino acids) of a protein encoded in a translation region of the base sequence are set forth in the SEQ ID No. 2 in accompanying SEQUENCE LISTING.

We describe proteins having the amino acid sequence represented by SEQ ID No. 1 and proteins having an amino acid sequence obtained by modifying the amino acid sequence represented by SEQ ID No. 1 by deletion, substitution, or addition of one or more amino acids. The degree of the deletion, the substitution, or the addition of amino acids is not limited so far as it does not cause an organic anion transport activity to be lost, and the number of amino acids to be involved may typically be 1 to about 110, preferably from 1 to about 55. The protein has typically from 1 to 80%, preferably from 1 to 90% amino acid sequence homology with the amino acid sequence represented by SEQ ID No. 1.

We describe a gene having the base sequence represented by SEQ ID No. 2 and a gene containing a DNA capable of hybridizing with a DNA having the base sequence represented by SEQ ID No. 2 under stringent conditions. The DNA capable of the hybridization is not limited so far as a protein encoded by the DNA is capable of transporting an organic anion. Such DNA has typically 70% or more, preferably 80% or more base sequence homology with the base sequence represented by SEQ ID No. 2. Examples of the DNA include such as variant genes found in nature, variant genes modified artificially, xenobiotics-derived homologous genes.

The hybridization under stringent conditions can be practiced typically by performing hybridization in 5 × SSC or in a hybridization solution having a salt concentration equivalent to 5 × SSC under a temperature condition of from 37 to 42°C for about 12 hours, followed by a pre-washing, when required, using 5 × SSC or a solution having a salt concentration equivalent to 5 x SSC, and then washing in 1 × SSC or in a solution having a salt concentration equivalent to 1 x SSC.

The liver-specific organic anion transporter gene which selectively transports the sulfate conjugate in the invention can be isolated and obtained by a screening using an organ, a tissue, or a cultured cell of a proper mammal as a gene source. Examples of the mammal include non-human animals such as a dog, a cow, a horse, a goat, a sheep, a monkey, a pig, a rabbit, a rat, and a mouse as well as a human.

The screening and isolation of a gene may preferably be performed by a homology cloning method and the like.

For instance, an mRNA (poly(A)⁺RNA) is prepared by using a mouse or the human liver as a gene source. By constructing a cDNA library therefrom, it is possible to obtain a clone containing a cDNA of the OAT7 gene by screening the cDNA library using a probe corresponding to a sequence similar to OAT7 (for example, GenBankTM/EBI/DDBJ accession No. AA705512) which is obtainable by a search in the EST (expressed sequence tag) database.

By determining a base sequence of the thus-obtained cDNA by an ordinary method and analyzing a translation region, it is possible to determine a protein encoded by the cDNA, i.e., an amino acid sequence of OAT7.

It is possible to verify whether or not the obtained cDNA is a cDNA of the organic anion transporter gene which selectively transports a sulfate conjugate, i.e., whether or not the gene product encoded by the cDNA is the transporter which selectively transports a sulfate conjugate, by the following process. That is, after expressing an RNA (cRNA; encapsulated) which is prepared from the cDNA of the obtained OAT7 gene and complementary thereto by introducing into an oocyte, an ability to transport (uptake) a sulfate conjugate into cells can be recognized by measuring an uptake of substrate into cells by an ordinary uptake experiment (Kanai and Hediger, Nature, Vol. 360, pp. 467-471, 1992) using a proper sulfate conjugate as the substrate.

By synthesizing an OAT7 protein using the RNA (cRNA) which is prepared from the cDNA of the obtained OAT7 gene and complementary thereto by the in vitro translation method (Hediger et al., Biochem. Biophys. Acta, Vol. 1064, p. 360, 1991), it is possible to examine the size, absence or presence of sugar addition, and so forth of the protein by way of an electrophoresis.

Further, it is possible to examine the expressed cell in terms of characteristics of OAT7, such as substrate selectivity and pH dependency through the same uptake experiment.

By screening a proper cDNA library or a genomic DNA library prepared from a different gene source with the use of the cDNA of the thus-obtained OAT7 gene, it is possible to isolate a homologous gene, a chromosome gene, and the like derived from a different tissue or organism.

Also, by searching various gene protein databases using the disclosed base sequence of the gene of the invention or amino acid sequence of its gene product, it is possible to identify a homologous gene, a chromosome gene, and the like derived from a different tissue or organism.

Further, by using a synthetic primer designed based on the disclosed information of the base sequence (the base sequence represented by SEQ ID No. 2 or a part thereof) of the gene of the invention, it is possible to isolate a gene from a cDNA library or a genomic DNA library by an ordinary PCR (Polymerase Chain Reaction) method.

The DNA library such as the cDNA library and the genomic library can be prepared according to the method disclosed in, for example, Molecular cloning, Sambrook, J., Fritsh, E. F., and Manitis, T., published in 1989 by Cold Spring Harbor Press. Alternatively, a commercially available library may be used, if any.

It is possible to produce the liver-specific organic anion transporter (OAT7) which selectively transports a sulfate conjugate by, for example, a gene recombinant technology using the cDNA encoding OAT7. For instance, incorporating the DNA (cDNA or the like) encoding OAT7 into a proper expression vector, and the obtained recombinant DNA can be introduced into a proper host cell. Examples of an expression system (host-vector system) for producing a polypeptide include such as expression systems of bacteria, yeast, an insect cell, and a mammalian cell. Among those examples, it is preferable to use the insect cell or the mammalian cell when it is desired to obtain a functional protein.

For instance, in the case of expressing a polypeptide with the use of the mammalian cell, the DNA encoding the liver-specific organic anion transporter OAT7 which selectively transports a sulfate conjugate is inserted downstream of a proper promoter (for example, a cytomegalovirus promoter, an SV40 promoter, an LTR promoter, an elongation la promoter, and so forth) in a proper expression vector (for example, an adenovirus based vector, a retrovirus based vector, a papilloma virus vector, a vaccinia virus vector, an SV40 based vector) to construct the expression vector. Next, a proper animal cell is transformed in the thus-obtained expression vector, and the transformant is cultured in a proper medium, to obtain the desired polypeptide. Examples of the host mammalian cell include such as a cell strain of a mouse S2 cell, a monkey COS-7 cell, a Chinese hamster CHO cell, or a human HeLa cell.

- As the DNA encoding the liver-specific organic anion transporter OAT7 which selectively transports sulfate conjugates, for example, the cDNA having the base sequence represented by SEQ ID No. 2 can be used and in addition, without limited to the above-described cDNA sequence, a DNA designed to correspond to the amino acid sequence can be used as a DNA encoding the polypeptide. In this case, each of 1 to 6 kinds of codons is known to encode one amino acid, and, though codons to be used can be decided arbitrarily, it is possible to design a sequence having a higher expression efficiency is achieved in view of a frequency of usage of each codons by the host to be used for the expression. The DNA having the thus-designed base sequence is obtainable by DNA chemical synthesis, fragmentation and binding of the above-described cDNA, partial modification of the base sequence, and the like. It is possible to perform the artificial partial modification and mutagenesis of base sequence by the site specific mutagenesis (Mark, D. F. et al., Proceedings of National Academy of Sciences, Vol. 81, page 5662, 1984) and the like, using a primer constituted of synthetic oligonucleotides encoding the desired modification.

We also describe a nucleotide including a partial sequence of 14 or more consecutive bases, preferably 20 or more bases of the base sequence represented by SEQ ID No. 2 or a complementary sequence thereof.

The nucleotide can be used as a probe for detecting a gene encoding a protein capable of selectively transporting a sulfate conjugate and as a primer for obtaining a gene encoding the protein or a gene encoding a protein having a high homology with the protein and, also can be used for modulating the expression of a gene encoding a protein capable of selectively transporting a sulfate conjugate by the antisense chain or the like.

By using the liver-specific organic anion transporter which selectively transports a sulfate conjugate or a polypeptide having an immunological equivalence thereto, its antibody can be obtained. The antibody is usable for detection and purification of the liver-specific organic anion transporter which selectively transports a sulfate conjugate. The antibody can be prepared by using the liver-specific organic anion transporter which selectively transports a sulfate conjugate, a fragment thereof, or a synthetic peptide having a partial sequence thereof as an antigen. A polyclonal antibody can be prepared by an ordinary method of inoculating a host animal (for example, rat or rabbit) with the antigen and then collecting an immune serum, while a monoclonal antibody can be prepared by an ordinary technique such as the hybridoma method.

The liver-specific organic anion transporter OAT7 which selectively transports a sulfate conjugate, a gene thereof, and an expressed cell thereof are usable for an in vitro experiment on permeation efficiency of a cell membrane in which OAT7 is present and a site in which OAT7 is expected to be present. That is, with the use of the protein of the invention, it is possible to detect a substrate action of a test substance on the ability of the protein of selectively transporting a sulfate conjugate.

Also, the liver-specific organic anion transporter OAT7 which selectively transports a sulfate conjugate, the gene thereof, and the expressed cell thereof are usable for development of a compound which efficiently permeates through the cell membrane in which OAT7 is present and the site in which OAT7 is expected to be present.

The liver-specific organic anion transporter OAT7 which selectively transports a sulfate conjugate, the gene thereof, and the expressed cell thereof are usable for development of an inhibitor (OAT7-specific inhibitor and the like) which suppresses the compound transported by OAT7 from permeating through the cell membrane and the site in which OAT7 is expected to be present.

For instance, an experiment of uptake of a substance (for example, a sulfate conjugate of a steroid hormone) transported by OAT7 can be conducted by using an oocyte into which the human OAT7 gene cRNA was injected. This experiment is conducted under the presence of various drugs to measure an absorption speed of the substance transported by OAT7. The drug which reduces the absorption speed of the substance transported by OAT7 is obtained as an inhibitor for an ability of OAT7 of selectively transporting the sulfate conjugate.

Also, The liver-specific organic anion transporter OAT7 which selectively transports a sulfate conjugate, the gene thereof, and the expressed cell thereof are usable for an in vitro experiment on drug interaction at the cell membrane in which OAT7 is present and the site in which OAT7 is expected to be present.

With the use of the liver-specific organic anion transporter which selectively transports a sulfate conjugate, its specific antibody, and its function-activating substance or function-inhibiting substance, it is possible to alter the ability of the protein of selectively transporting a sulfate conjugate and to modify pharmacokinetics of a drug, toxic substance, xenobiotics, or steroid hormone transported by the transporter of the invention.

The transporter of the invention can be produced by the above-described gene recombinant technique, and it is possible to use the transporter as an internal transfer activating agent of a sulfate conjugate. Also, it is possible to develop the function-activating substance or function-inhibiting substance of the liver-specific organic anion transporter which selectively transports a sulfate conjugate by way of an experiment using the organic anion transporter of the invention and expressed cell thereof, and, with the use of the drugs, it is possible to modify pharmacokinetics of a drug, toxic substance, xenobiotics, or steroid hormone transported by the transporter of the invention.

Further, by modifying the pharmacokinetics of the drug transported by the transporter of the invention, it is possible to reduce a drug's side effect and a toxic action of xenobiotics. Also, by modifying the pharmacokinetics of the compound transported by the transporter of the invention, it is possible to protect the hepatic cells from drugs and xenobiotics.

### EXAMPLES

Though the present invention will hereinafter be described in more detail in conjunction with examples, however, the invention is not limited by the examples.

In the examples, operations are performed in accordance with the method described in Molecular cloning, written by Sambrook, J., Fritsh, E. F., and Manitis, T., published by Cold Spring Harbor Press in 1989 unless otherwise described or, in the case of using commercially available agents and kits, in accordance with instructions of the products.

### Example 1: Cloning of a human cDNA of the liver-specific organic anion transporter gene selectively transporting a sulfate conjugate

### (1) Isolation of a human cDNA of the liver-specific organic anion transporter OAT7 which selectively transports a sulfate conjugate and preparation of a cRNA

A sequence corresponding to a sequence similar to OAT1 (GenBankTM/EBI/DDBJ accession No. AA705512) which was obtained by a search in the EST (expressed sequence tag) database using a base sequence of a translation region of OAT1 was amplified by a reverse transcription-PCR using a human liver-derived poly(A)⁺RNA as a template, and the amplification product was labeled with ³²P-dCTP to be used as a probe for screening a human liver cDNA library.

The cDNA library was created from the human liver-derived poly(A)⁺RNA by using a cDNA synthesize kit (product name: Superscript Choice System; manufactured by Gibco Industries, Inc.) and then incorporated into a limited enzyme EcoRI cut section of phage vector λ ZipLox (manufactured by Gibco Industries, Inc.). The hybridization by the probe with the ³²P-dCTP labeling was performed overnight in a hybridization solution of 37°C, and a filter membrane was washed with 0.1 × SSC/0.1% SDS at 37°C. Used as the hybridization solution was a buffer solution of pH 6.5 containing 5 × SSC, 3 × Denhard's solution, 0.2% SDS, 10% dextran sulfate, 50% formamide, 0.01% Abtiform B (manufactured by Sigma-Aldrich Japan K.K.) (antifoaming agent), 0.2 mg/ml modified sermon sperm DNA, 2.5 mM sodium pyrophosphate, and 25 mM MES. A cDNA portion of the λ ZipLox phage into which the cDNA was incorporated was incorporated into a plasmid pZL1.

A base sequence of a cDNA of the thus-obtained clone, i.e., of the clone containing the cDNA of human OAT7, was determined by a dye terminator cycle sequencing method (Applied Biosystems) using a synthetic primer for the base sequence determination.

Thus, a base sequence of the human OAT7 gene was obtained. Further, through an analysis of the base sequence of the cDNA by an ordinary method, a translation region of the cDNA and an amino acid sequence of human OAT7 encoded in the translation region were determined.

The sequences are set forth in SEQ ID No. 2 in attached SEQUENCE LISTING.

OAT7 shared a 42% amino acid sequence homology with the human organic anion transporter OAT1, a 35% amino acid sequence homology with the rat organic anion transporter OAT2, a 41% amino acid sequence homology with the human organic anion transporter OAT3, and a 46% amino acid sequence homology with the human organic anion transporter OAT4.

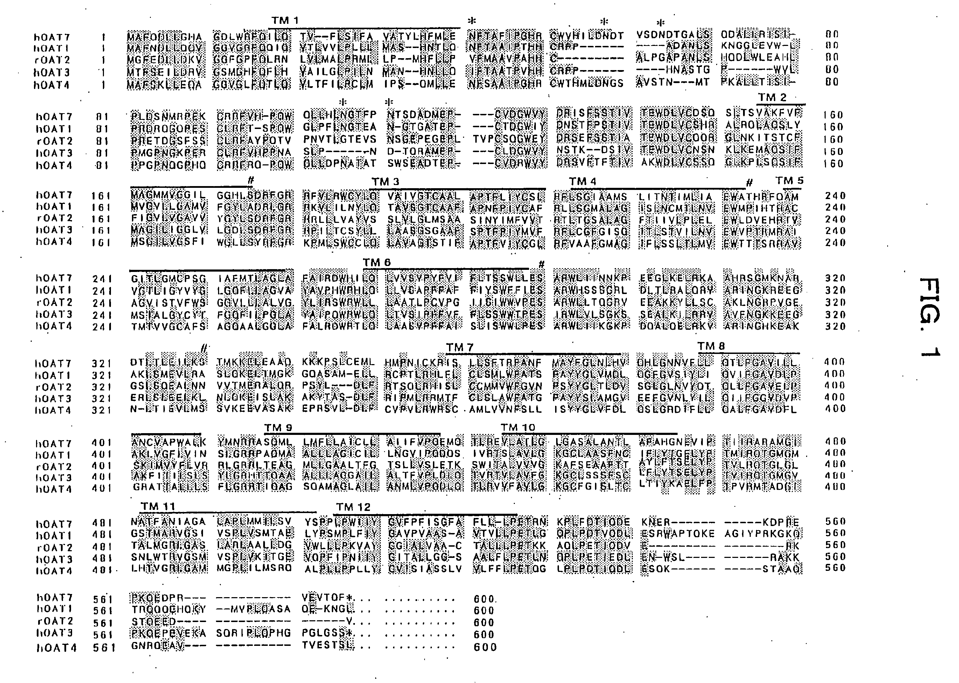

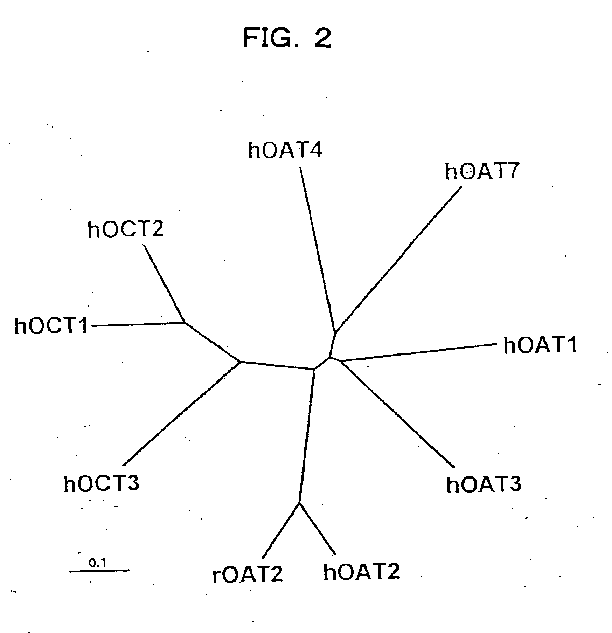

Comparison among the amino acid sequences of OAT7, human OAT1, rat OAT2, human OAT3, and human OAT4 is shown in Fig. 1. In Fig. 1, an expected membrane-spanning section is indicated by a line. Also, an expected glycosylation site and an expected C-kinase-dependent phosphorylation site are indicated by * and #, respectively. Also, shown in Fig. 2 is a phylogenetic tree based on amino acid sequences of human OAT7, human OAT1, rat OAT2, human OAT2, human OAT3, human OAT4, and amino acid sequences of human OCT1, human OCT2, and human OCT3, which are organic cation transporters having a structure similar to that of OAT.

As a result of an analysis of the amino acid sequence of OAT7 by the Top Pred2 algorithm (von Heijne, G et al., J. Mol. Biol., Vol. 225, page 487, 1992; Cserzo, M. et al., Protein Eng., Vol. 10, page 673, 1997), 12 membrane-spanning domains were expected as shown in Fig. 1. Also, a section considered to be the glycosylation site was found in the first hydrophilic loop, and a section considered to be the protein kinase C-dependent phosphorylation section was found in each of the second, the fourth, and the sixth hydrophilic loops.

### (2) Identification of a human OAT7 chromosome gene and determination of an exon-intron structure

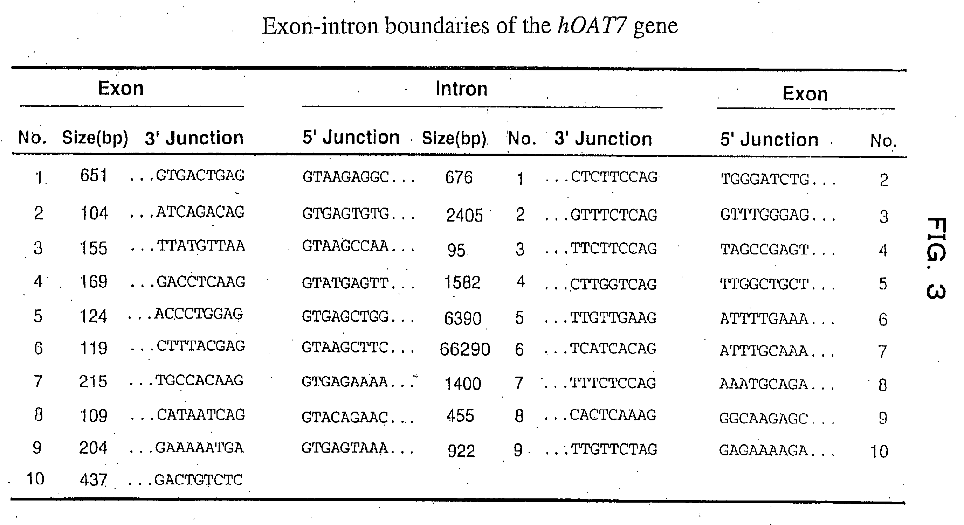

As a result of a search in NCBI human genome base sequence database using the base sequence of the cDNA of human OAT7, it was revealed that Contig RP11-151E18 (Accession No. AP002367) mapped on the eleventh chromosome includes a sequence identical with that of the base sequence of human OAT7. By comparing the base sequence of the Contig with the base sequence of the cDNA of human OAT7, an exon-intron structure of human OAT7 was determined. A result thereof is shown in Fig. 3.

### (3) Expression of an OAT7 gene in various human tissues (analysis by northern blotting)

A total length of the cDNA of OAT7 was taken out by EcoRI and labeled with ³²P-dCTP to be used as a probe, and then hybridization was conducted by the probe using Human Blot (manufactured by Clontech) and in accordance with the attached protocol.

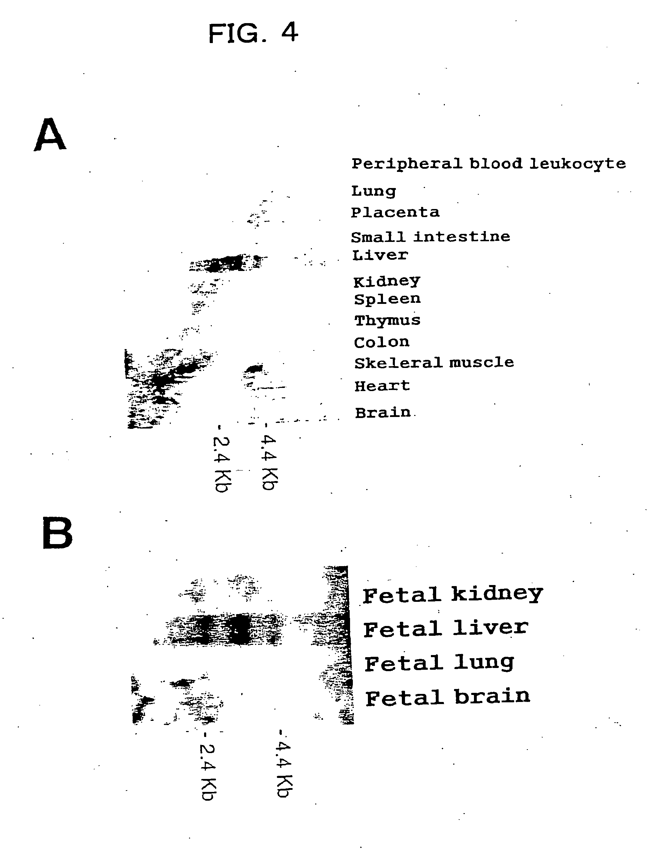

As a result of the northern blotting (Fig. 4A) a band was detected near 2.4 kb, 3.0 kb, and 4.4 kb only in the liver. Also, a comparison among the fetal liver, the fetal kidney, the fetal lung, and the fetal brain was conducted by the northern blotting of the fetal tissues, and the expression was detected only in the fetal liver (Fig. 4B). The size of the band was the same as that of the adult liver.

### (4) Expression of an OAT7 protein in the human liver

An antibody specific to a synthetic oligopeptide [CKQEDPRVEVTQ] corresponding to 542 to 552 of human OAT7 was prepared in accordance with the method of Altman et al. (Altman et al., Proc. Natl. Acad. Sci. U.S.A., Vol. 81, pages 2176 to 2180, 1984). A cysteine residue at N-terminus was introduced for the purpose of KLH conjugation.

Human liver total proteins and human liver protein membrane fractions (both of which were purchased from Biochain Inc.) were subjected to an electrophoresis using an SDS-polyacrylamide gel, followed by blotting on a Hybond-P PVDV transfer membrane and a treatment with a peptide affinity purified anti-OAT7 antibody (1 : 100).

As a result, a band near 50 kDa was detected with each of the human liver total proteins and the human liver protein membrane fractions by the anti-OAT7 antibody as shown in Fig. 5A.

### (5) Immunohistochemical analysis of the OAT7 protein in the human liver

After treating a human liver paraffin section (purchased from Biochain Inc.) with the peptide affinity purified anti-OAT7 antibody (1 : 100) according to an ordinary method, color development was performed with diaminobenzidine. Also, for the purpose of studying specificity of the staining, an experiment of treating with the peptide affinity purified anti-OAT7 antibody (1 : 100) under the presence of a 200 µg/ml of antigen peptide was performed.

As a result, staining was observed in the hepatic cells as shown in Fig. 5C. This staining was not detected in the case of applying the anti-OAT7 antiserum under the presence of the antigen peptide, showing the specificity of the staining (Fig. 5E). Further, through an observation with a highly magnified, it was revealed that the OAT7 protein exists in the basolateral plasma membrane of the hepatic cell (Fig. 5D).

### Example 2: Characterization of the liver-specific organic anion transporter OAT7 selectively transporting a sulfate conjugate

### (1) Transport activity of OAT7

After expressing the human OAT7 gene cRNA in an oocyte, [³H]estrone-sulfate conjugate uptake and [³H]dehydroepiandrosterone-sulfate conjugate uptake were measured.

After injecting 20 ng of the human OAT7 gene cRNA into the oocyte to be expressed, a 3-days culture was performed.

A substrate uptake experiment was performed on the oocyte into which the human OAT7 gene cRNA was injected and a control oocyte into which water was injected, using the [³H]estrone-sulfate conjugate and the [³H]dehydroepiandrosterone-sulfate conjugate as the substrates in accordance with the method of Kanai et al. (Kanai and Hediger, Nature, Vol. 360, pp. 467 to 471, 1992) as described below. After leaving the oocyte in an ND96 solution (96 mM sodium chloride, 2 mM potassium chloride, 1.8 mM calcium chloride, 1 mM magnesium chloride, 5 mM HEPES, pH7.4) containing the [³H]estrone-sulfate conjugate (50 nM) and the [³H]dehydroepiandrosterone-sulfate conjugate (100 nM) as a substrate for 60 minutes, and an uptake ratio of the substrate was measured by way of counting radioactivity taken up into the cells.

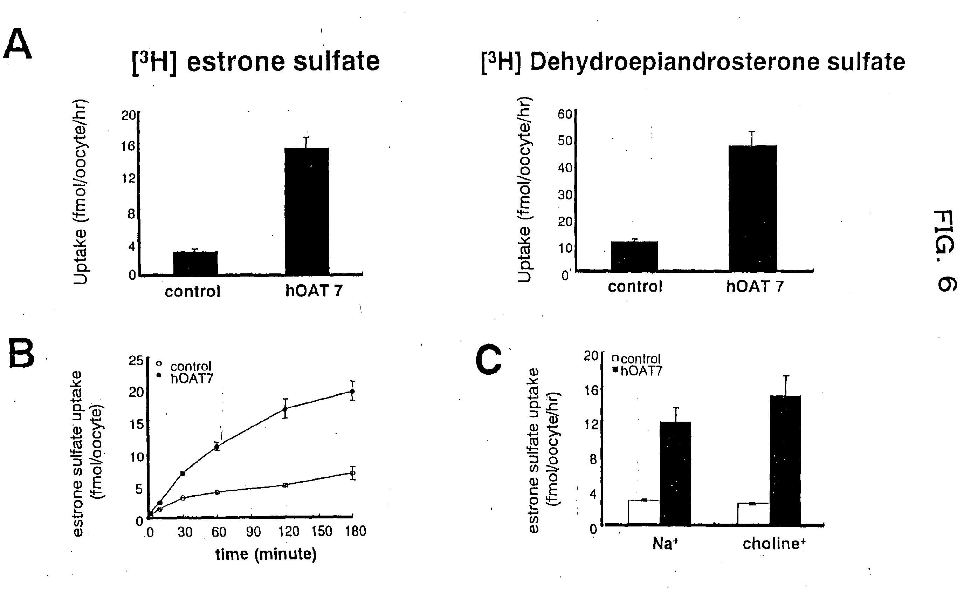

As a result (Fig. 6A), the [³H]estrone-sulfate conjugate uptake and the [³H]dehydroepiandrosterone-sulfate conjugate uptake by the oocytes in which OAT7 was expressed was significantly greater than that by the control oocyte into which water was injected.

### (2) Time dependency of the OAT7 transport activity

Time passage of the substrate uptake by the oocyte into which the human OAT7 gene cRNA was injected and a control oocyte into which water was injected was observed using the [³H]estrone-sulfate conjugate (50 nM) as the substrate and in accordance with the method of Example 2(1).

As a result (Fig. 6B), it was revealed that the [³H]estrone-sulfate conjugate uptake by way of OAT7 increases substantially linearly in a time dependent manner till the passage of 120 minutes.

### (3) A sodium ion dependency of the OAT7 transport activity

The effect of salt added in the culture used in the [³H]estrone-sulfate conjugate (50 nM) uptake experiment were examined using an oocyte into which the human OAT7 gene cRNA was injected and a control oocyte into which water was injected.

The [³H]estrone-sulfate conjugate uptake experiment was conducted in accordance with the method described in Example 2(1) using the oocyte into which the human OAT7 gene cRNA was injected. In the experiment, a sodium ion free absorption solution (wherein 96 mM sodium chloride was substituted with 96 mM choline chloride) was used as an uptake solution in place of the standard absorption solution for the purpose of observing the effect of the sodium ion.

As a result (Fig. 6C), with the change of the extracellular sodium to choline, no effect was observed on the [³H]estrone-sulfate conjugate uptake (50 nM). From the result, it was revealed that OAT7 is a transporter which acts on the sodium ion in an independent manner.

### (4) Michaelis-Menten kinetics test on OAT7

A Michaelis-Menten kinetics test on the liver specific organic anion transporter OAT7 selectively transporting a sulfate conjugate was conducted. The Michaelis-Menten kinetics test on OAT7 was performed by examining changes in uptake ratio of the substrate [³H]estrone-sulfate conjugate and the [³H]dehydroepiandrosterone-sulfate conjugate depending on the concentrations of the substrate [³H]estrone-sulfate conjugate and the [³H]dehydroepiandrosterone-sulfate conjugate.

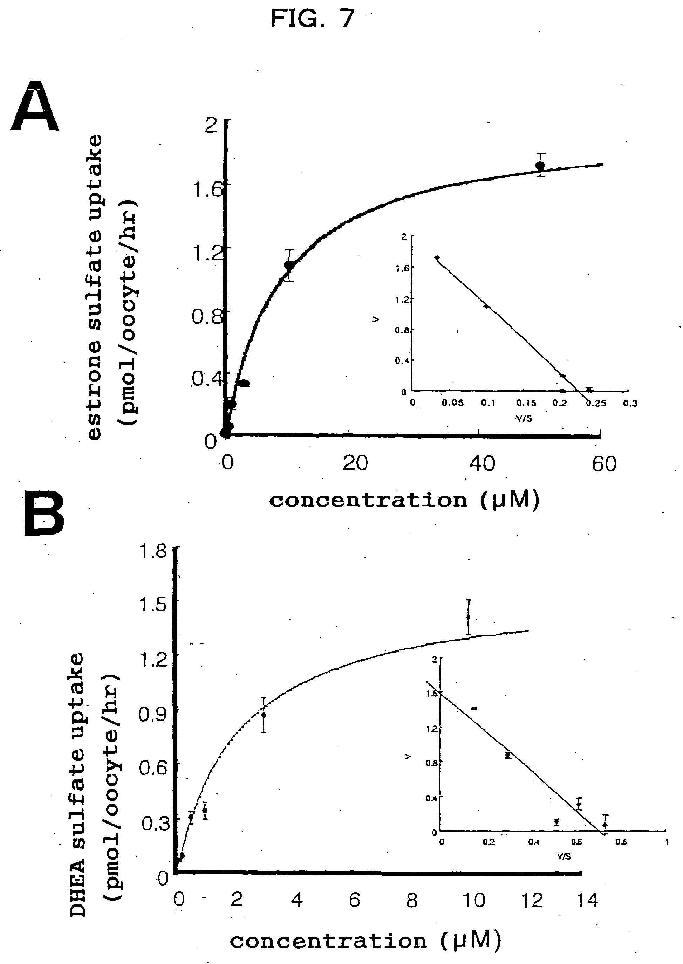

The [³H]estrone-sulfate conjugate uptake experiment and the [³H]dehydroepiandrosterone-sulfate conjugate uptake experiment were conducted using an oocyte into which the human OAT7 gene cRNA was injected and in accordance with the method described in Example 2(1). As a result (Figs. 7A and 7B), the obtained Km value was 8.7 ± 1.1 µM and 2.2 ± 0.3 µM (mean ± standard error), respectively.

### (5) Substrate selectivity of OAT7 (inhibition experiments by way of addition of various organic anions and organic cations)

In the [³H]estrone-sulfate conjugate uptake experiment using an oocyte into which the human OAT7 gene cRNA was injected, the effect of addition of various organic anions and organic cations on the system was examined.

The [³H]estrone-sulfate conjugate uptake experiment was conducted using the oocyte into which the human OAT7 gene cRNA was injected and in accordance with the method described in Example 2(1). In the experiment, [³H]estrone-sulfate conjugate (1 µM) uptake was measured under the presence and the absence of 100 µM of various compounds (unlabeled).

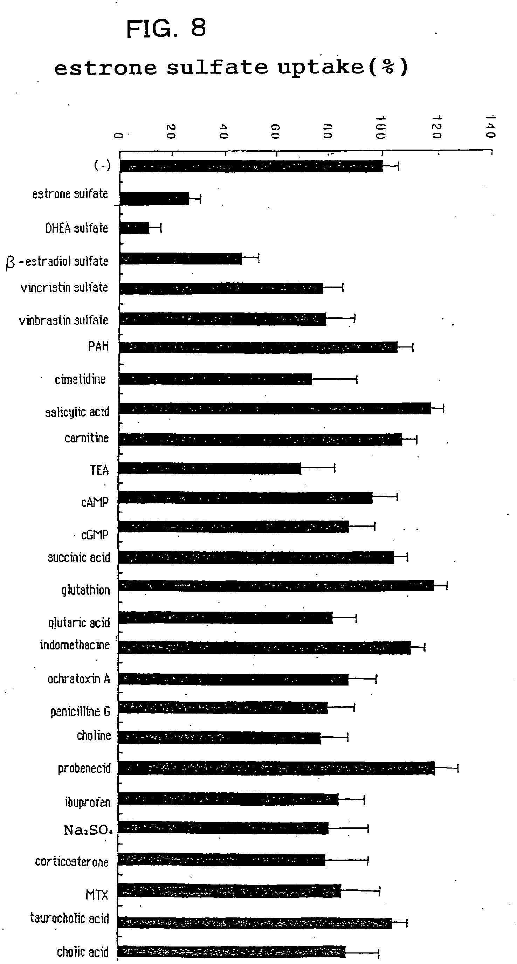

As a result, among the studied organic anions and organic cations shown in Fig. 8, the estrone-sulfate conjugate, the dehydroepiandrosterone-sulfate conjugate, and the β-estradiol sulfate conjugate exhibited a significant cys-inhibition effect. However, other compounds shown in Fig. 8 did not exhibit the significant cys-inhibition effect.

Since OAT7 is the organic anion transporter existing in the liver, effects of various organic anions which are considered to be transported in the hepatic cells on the [³H]estrone-sulfate conjugate uptake by way of OAT7 were examined.

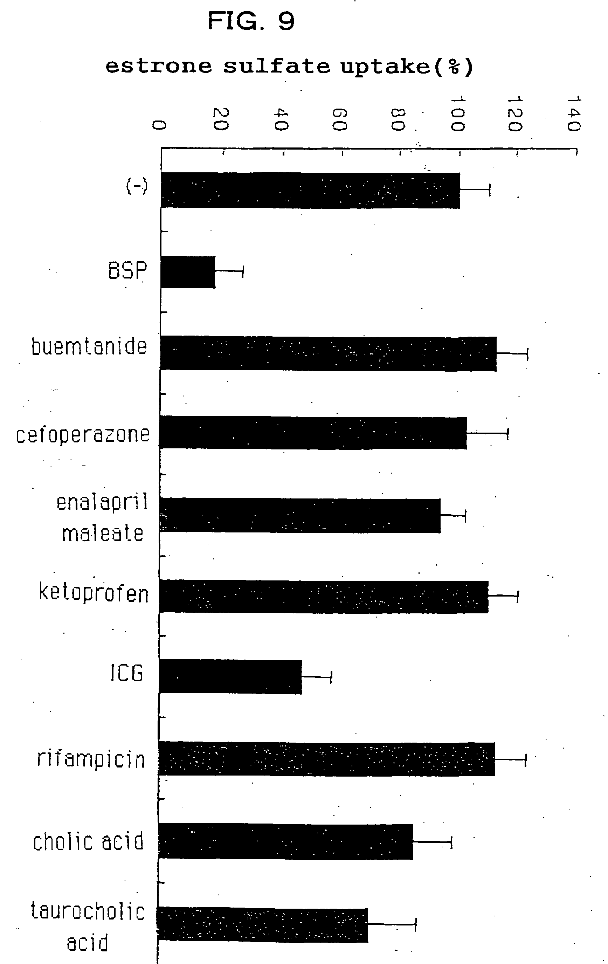

As a result, among the studied organic anions shown in Fig. 9, sulfobromophthalein (BSP) and indocyanin green (ICG) exhibited a significant cys-inhibition effect. However, other compounds shown in Fig. 9 did not exhibit the significant cys-inhibition effect.

In order to clarify which one of the sulfate conjugate, the glucuronide conjugate, the glutathione conjugate is accepted by OAT7, effects of various conjugate compounds on the [³H]estrone-sulfate conjugate uptake were examined.

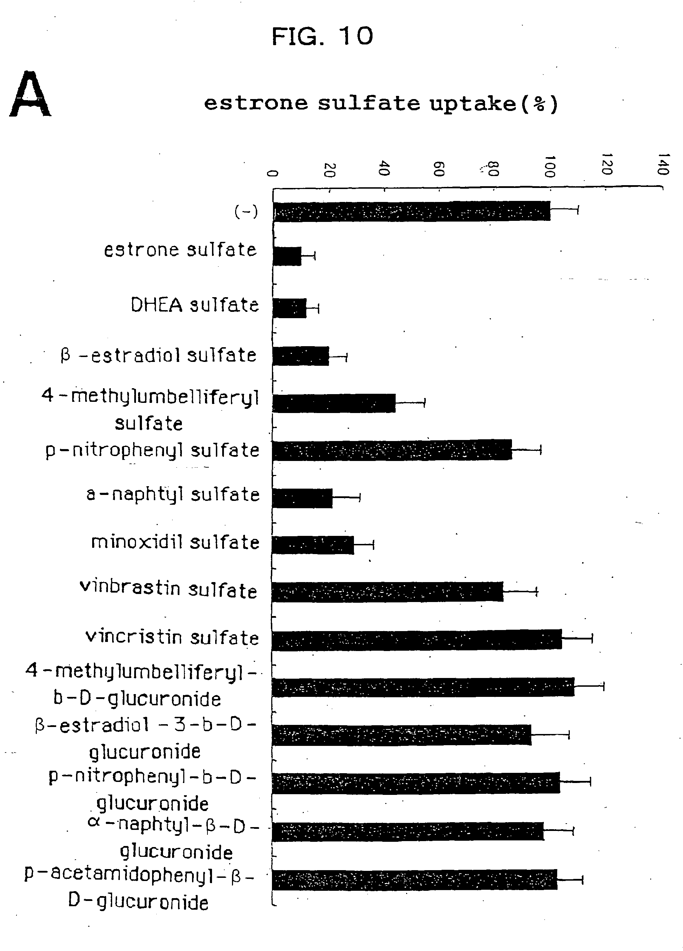

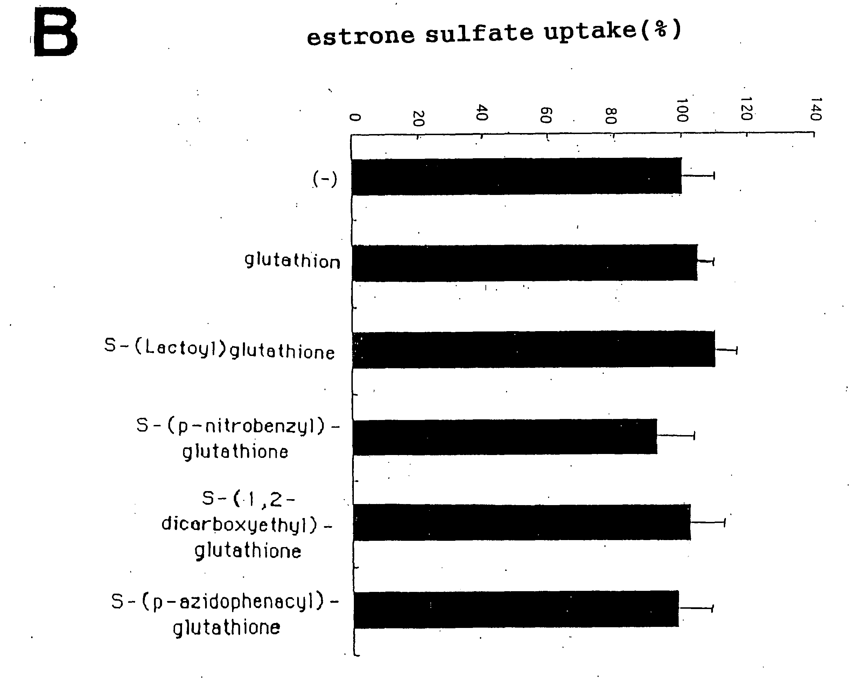

As a result, among the studied conjugate compounds shown in Figs. 10A and 10B, the estrone-sulfate conjugate, the dehydroepiandrosterone-sulfate conjugate, the p-nitrophenol-sulfate conjugate, the β-estradiol-sulfate conjugate, the 4-methylumbelliferyl sulfate conjugate, and the minoxidil-sulfate conjugate exhibited a significant cys-inhibition effect. However, other compounds shown in Figs. 10A and 10B, such as the glucuronide conjugate and the glutathione conjugate, did not exhibit the significant cys-inhibition effect.

### Industrial Applicability

The liver-specific organic anion transporter selectively transporting a sulfate conjugate and a gene thereof enable an in vitro investigation of a transport of a sulfate conjugate of a drug, xenobiotics, steroid hormone, and its analogous compound by the transporter in the liver and an in vitro assumption of pharmacokinetics of the drug, xenobiotics, steroid hormone, and analogous compound based on the investigation. Further, the present invention is considered to be useful for developments of drugs which efficiently permeate through the transporter. Also, the invention enables an in vitro assumption of drug interaction in which the transporter participates. Moreover, by altering the ability of the transporter of selectively transporting a sulfate conjugate, it is possible to use for developments of a method of modifying pharmacokinetics of a drug, toxic substance, xenobiotics, steroid hormone, and so forth, a method of reducing a drug's side effect and a toxic action of xenobiotics, and a method of protecting the hepatic cells from a drug and xenobiotics.

## Claims

1. A protein comprising the amino acid sequence represented by SEQ ID NO: 1 and being capable of selectively transporting a sulfate conjugate.

2. The protein according to claim 1, which is derived from a mammal.

3. The protein according to claim 2, which is derived from a human.

4. The protein according to any one of claims 1 to 3, which is derived from an organ, a tissue or a cultured cell.

5. A gene encoding a protein according to any one of claims 1 to 4 or a gene comprising a DNA comprising the base sequence represented by SEQ ID NO: 2 encoding a protein capable of selectively transporting a sulfate conjugate.

6. The gene according to claim 5, which is derived from a mammal.

7. The gene according to claim 6, which is derived from a human.

8. The gene according to any one of claims 5 to 7, which is derived from an organ, a tissue or a cultured cell.

9. A vector comprising a gene according to any one of claims 5 to 8.

10. The vector according to claim 9, which is an expression plasmid.

11. An isolated host cell transformed by a vector according to claim 9 or 10.

12. An antibody specific to the protein according to any one of claims 1 to 4.

13. A method for detecting whether a test substance is a substrate of a protein according to any one of claims 1 to 4 comprising detecting the effect of the test substance on the ability of the protein to selectively transport a sulfate conjugate, to thereby determine whether the test substance is a substrate of the protein.

14. A method of detecting whether a test substance is an inhibitor of a protein according to any one of claims 1 to 4 comprising detecting the effect of the test substance on the ability of the protein to selectively transport a sulfate conjugate, to thereby determine whether the test substance is an inhibitor of the protein.

15. A method of detecting a drug interaction with a substance via the protein, using the protein according to any one of claims 1 to 4.

16. Use of a protein according to any one of claims 1 to 4 which selectively transports a sulfate conjugate or an antibody specific thereto, in the manufacture of a medicament for modifying pharmacokinetics of a drug by altering the activity of said protein.

17. Use of a protein according to any one of claims 1 to 4 which selectively transports a sulfate conjugate or an antibody specific thereto, in the manufacture of a medicament for modifying pharmacokinetics of a toxic substance or xenobiotics by altering the activity of said protein.

18. Use of a protein according to any one of claims 1 to 4 which selectively transports a sulfate conjugate or an antibody specific thereto,
in the manufacture of a medicament for modifying pharmacokinetics of a steroid hormone by altering the activity of said protein.

19. Use according to claim 17 wherein the modification of pharmacokinetics reduces a drug's side effect or a toxic action of xenobiotics.

20. Use of a protein according to any one of claims 1 to 4 which or selectively transports a sulfate conjugate or an antibody specific thereto, in the manufacture of a medicament for protecting hepatic cells from a drug and xenobiotics by altering the activity of said protein.

## Patentansprüche

1. Protein, umfassend die durch SEQ ID NR. 1 dargestellte Aminosäuresequenz und fähig zum selektiven Transportieren eines Sulfat-Konjugats.

2. Protein nach Anspruch 1, welches von einem Säuger stammt.

3. Protein nach Anspruch 2, welches von einem Menschen stammt.

4. Protein nach einem der Ansprüche 1 bis 3, welches von einem Organ, einem Gewebe oder einer kultivierten Zelle stammt.

5. Gen, das für ein Protein nach einem der Ansprüche 1 bis 4 kodiert, oder Gen, das eine DNA enthält, umfassend die durch SEQ ID NR. 2 dargestellte Basensequenz, und das für ein Protein kodiert, welches zum selektiven Transportieren eines Sulfat-Konjugats fähig ist.

6. Gen nach Anspruch 5, welches von einem Säuger stammt.

7. Gen nach Anspruch 6, welches von einem Menschen stammt.

8. Gen nach einem der Ansprüche 5 bis 7, welches von einem Organ, einem Gewebe oder einer kultivierten Zelle stammt.

9. Vektor, umfassend ein Gen nach einem der Ansprüche 5 bis 8.

10. Vektor nach Anspruch 9, welcher ein Expressionsplasmid ist.

11. Isolierte Wirtszelle, die durch einem Vektor nach Anspruch 9 oder 10 transformiert ist.

12. Antikörper, der für das Protein nach einem der Ansprüche 1 bis 4 spezifisch ist.

13. Verfahren zum Nachweisen, ob eine Testsubstanz ein Substrat eines Proteins nach einem der Ansprüche 1 bis 4 ist, umfassend das Nachweisen der Wirkung der Testsubstanz auf die Fähigkeit des Proteins, ein Sulfat-Konjugat selektiv zu transportieren, um **dadurch** zu bestimmen, ob die Testsubstanz ein Substrat des Proteins ist.

14. Verfahren zum Nachweisen, ob eine Testsubstanz ein Inhibitor eines Proteins nach einem der Ansprüche 1 bis 4 ist, umfassend das Nachweisen der Wirkung der Testsubstanz auf die Fähigkeit des Proteins, ein Sulfat-Konjugat selektiv zu transportieren, um **dadurch** zu bestimmen, ob die Testsubstanz ein Inhibitor des Proteins ist.

15. Verfahren zum Nachweisen einer Wirkstoff-Interaktion mit einer Substanz über das Protein, unter Verwendung des Proteins nach einem der Ansprüche 1 bis 4.

16. Verwendung eines Proteins nach einem der Ansprüche 1 bis 4, welches ein Sulfat-Konjugat oder einen dafür spezifischen Antikörper selektiv transportiert, in der Herstellung eines Medikaments zum Modifizieren der Pharmakokinetiken eines Wirkstoffs, indem die Aktivität des Proteins verändert wird.

17. Verwendung eines Proteins nach einem der Ansprüche 1 bis 4, welches ein Sulfat-Konjugat oder einen dafür spezifischen Antikörper selektiv transportiert, in der Herstellung eines Medikaments zum Modifizieren der Pharmakokinetiken einer toxischen Substanz oder von Xenobiotika, indem die Aktivität des Proteins verändert wird.

18. Verwendung eines Proteins nach einem der Ansprüche 1 bis 4, welches ein Sulfat-Konjugat oder einen dafür spezifischen Antikörper selektiv transportiert, in der Herstellung eines Medikaments zum Modifizieren der Pharmakokinetiken eines Steroidhormons, indem die Aktivität des Proteins verändert wird.

19. Verwendung nach Anspruch 17, wobei die Modifikation der Pharmakokinetiken eine Nebenwirkung des Wirkstoffs oder eine toxische Wirkung von Xenobiotika reduziert.

20. Verwendung eines Proteins nach einem der Ansprüche 1 bis 4, welches ein Sulfat-Konjugat oder einen dafür spezifischen Antikörper selektiv transportiert, in der Herstellung eines Medikaments zum Schützen hepatischer Zellen vor einem Wirkstoff und vor Xenobiotika, indem die Aktivität des Proteins verändert wird.

## Revendications

1. Protéine comprenant la séquence d'acides aminés représentée par SEQ ID NO :1 et étant capable de transporter sélectivement un conjugué sulfate.

2. Protéine selon la revendication 1, étant issue d'un mammifère.

3. Protéine selon la revendication 2, étant issue d'un humain.

4. Protéine selon l'une quelconque des revendications 1 à 3, étant issue d'un organe, d'un tissu ou d'une cellule en culture.

5. Gène codant pour une protéine selon l'une quelconque des revendications 1 à 4 ou gène comprenant un ADN comprenant la séquence de base représentée par SEQ ID NO :2, codant pour une protéine capable de transporter sélectivement un conjugué sulfate.

6. Gène selon la revendication 5, étant issu d'un mammifère.

7. Gène selon la revendication 6, étant issu d'un humain.

8. Gène selon l'une quelconque des revendications 5 à 7, étant issu d'un organe, d'un tissu ou d'une cellule en culture.

9. Vecteur comprenant un gène selon l'une quelconque des revendications 5 à 8.

10. Vecteur selon la revendication 9, étant un plasmide d'expression.

11. Cellule hôte isolée transformée par un vecteur selon la revendication 9 ou 10.

12. Anticorps spécifique de la protéine selon l'une quelconque des revendications 1 à 4.

13. Méthode pour détecter si une substance de test est un substrat d'une protéine selon l'une quelconque des revendications 1 à 4 comprenant la détection de l'effet de la substance de test sur la capacité de la protéine à transporter sélectivement un conjugué sulfate, pour déterminer ainsi si la substance de test est un substrat de la protéine.

14. Méthode pour détecter si une substance de test est un inhibiteur d'une protéine selon l'une quelconque des revendications 1 à 4, comprenant la détection de l'effet de la substance de test sur la capacité de la protéine à transporter sélectivement un conjugué sulfate, pour déterminer ainsi si la substance de test est un inhibiteur de la protéine.

15. Méthode de détection d'une interaction de médicament avec une substance via la protéine, en utilisant la protéine selon l'une quelconque des revendications 1 à 4.

16. Utilisation d'une protéine selon l'une quelconque des revendications 1 à 4 transportant sélectivement un conjugué sulfate ou un anticorps spécifique de celui-ci, dans la préparation d'un médicament pour la modification pharmacocinétique d'un médicament en modifiant l'activité de ladite protéine.

17. Utilisation d'une protéine selon l'une quelconque des revendications 1 à 4 transportant sélectivement un conjugué sulfate ou un anticorps spécifique de celui-ci, dans la préparation d'un médicament pour la modification pharmacocinétique d'une substance toxique ou de xénobiotiques en modifiant l'activité de ladite protéine.

18. Utilisation d'une protéine selon l'une quelconque des revendications 1 à 4 transportant sélectivement un conjugué sulfate ou un anticorps spécifique de celui-ci, dans la préparation d'un médicament pour la modification pharmacocinétique d'une hormone stéroïde en modifiant l'activité de ladite protéine.

19. Utilisation selon la revendication 17, dans laquelle la modification de pharmacocinétique réduit un effet secondaire du médicament ou une action toxique de xénobiotiques.

20. Utilisation d'une protéine selon l'une quelconque des revendications 1 à 4 transportant sélectivement un conjugué sulfate ou un anticorps spécifique de celui-ci, dans la préparation d'une médicament pour protéger des cellules hépatique d'un médicament et de xénobiotiques en modifiant l'activité de ladite protéine.


### SEQUENCE LISTING

<110> Japan Science and Technology Agency
<120> TRANSPORTER SELECTIVELY TRANSPORTING SULFATE CONJUGATE AND ITS GENE
<130> JA904203
<150> JP2002-070985
   <151> 2002-3-14
<160> 3
<170> Patent In version 3.1
<210> 1
   <211> 553
   <212> PRT
   <213> Homo sapiens
<400> 1
   Met Ala Phe Gln Asp Leu Leu Gly His Ala Gly Asp Leu Trp Arg Phe
   1 5 10 15
   Gln Ile Leu Gln Thr Val Phe Leu Ser Ile Phe Ala Val Ala Thr Tyr
   20 25 30
   Leu His Phe Met Leu Glu Asn Phe Thr Ala Phe Ile Pro Gly His Arg
   35 40 45
   Cys Trp Val His Ile Leu Asp Asn Asp Thr Val Ser Asp Asn Asp Thr
   50 55 60
   Gly Ala Leu Ser Gln Asp Ala Leu Leu Arg Ile Ser Ile Pro Leu Asp
   65 70 75 80
   Ser Asn Met Arg Pro Glu Lys Cys Arg Arg Phe Val His Pro Gln Trp
   85 90 95
   Gln Leu Leu His Leu Asn Gly Thr Phe Pro Asn Thr Ser Asp Ala Asp
   100 105 110
   Met Glu Pro Cys Val Asp Gly Trp Val Tyr Asp Arg Ile Ser Phe Ser
   115 120 125
   Ser Thr Ile Val Thr Glu Trp Asp Leu Val Cys Asp Ser Gln Ser Leu
   130 135 140
   Thr Ser Val Ala Lys Phe Val Phe Met Ala Gly Met Met Val Gly Gly
   145 150 155 160
   Ile Leu Gly Gly His Leu Ser Asp Arg Phe Gly Arg Arg Phe Val Leu
   165 170 175
   Arg Trp Cys Tyr Leu Gln Val Ala Ile Val Gly Thr Cys Ala Ala Leu
   180 185 190
   Ala Pro Thr Phe Leu Ile Tyr Cys Ser Leu Arg Phe Leu Ser Gly Ile
   195 200 205
   Ala Ala Met Ser Leu Ile Thr Asn Thr Ile Met Leu Ile Ala Glu Trp
   210 215 220
   Ala Thr His Arg Phe Gln Ala Met Gly Ile Thr Leu Gly Met Cys Pro
   225 230 235 240
   Ser Gly Ile Ala Phe Met Thr Leu Ala Gly Leu Ala Phe Ala Ile Arg
   245 250 255
   Asp Trp His Ile Leu Gln Leu Val Val Ser Val Pro Tyr Phe Val Ile
   260 265 270
   Phe Leu Thr Ser Ser Trp Leu Leu Glu Ser Ala Arg Trp Leu Ile Ile
   275 280 285
   Asn Asn Lys Pro Glu Glu Gly Leu Lys Glu Leu Arg Lys Ala Ala His
   290 295 300
   Arg Ser Gly Met Lys Asn Ala Arg Asp Thr Leu Thr Leu Glu Ile Leu
   305 310 315 320
   Lys Ser Thr Met Lys Lys Glu Leu Glu Ala Ala Gln Lys Lys Lys Pro
   325 330 335
   Ser Leu Cys Glu Met Leu His Met Pro Asn Ile Cys Lys Arg Ile Ser
   340 345 350
   Leu Leu Ser Phe Thr Arg Phe Ala Asn Phe Met Ala Tyr Phe Gly Leu
   355 360 365
   Asn Leu His Val Gln His Leu Gly Asn Asn Val Phe Leu Leu Gln Thr
   370 375 380
   Leu Phe Gly Ala Val Ile Leu Leu Ala Asn Cys Val Ala Pro Trp Ala
   385 390 395 400
   Leu Lys Tyr Met Asn Arg Arg Ala Ser Gln Met Leu Leu Met Phe Leu
   405 410 415
   Leu Ala Ile Cys Leu Leu Ala Ile Ile Phe Val Pro Gln Glu Met Gln
   420 425 430
   Thr Leu Arg Glu Val Leu Ala Thr Leu Gly Leu Gly Ala Ser Ala Leu
   435 440 445
   Ala Asn Thr Leu Ala Phe Ala His Gly Asn Glu Val Ile Pro Thr Ile
   450 455 460
   Ile Arg Ala Arg Ala Met Gly Ile Asn Ala Thr Phe Ala Asn Ile Ala
   465 470 475 480
   Gly Ala Leu Ala Pro Leu Met Met Ile Leu Ser Val Tyr Ser Pro Pro
   485 490 495
   Leu Pro Trp Ile Ile Tyr Gly Val Phe Pro Phe Ile Ser Gly Phe Ala
   500 505 510
   Phe Leu Leu Leu Pro Glu Thr Arg Asn Lys Pro Leu Phe Asp Thr Ile
   515 520 525
   Gln Asp Glu Lys Asn Glu Arg Lys Asp Pro Arg Glu Pro Lys Gln Glu
   530 535 540
   Asp Pro Arg Val Glu Val Thr Gln Phe
   545 550
<210> 2
   <211> 2342
   <212> DNA
   <213> Homo sapiens
<220>
   <221> CDS
   <222> (250)..(1911)
   <223> OAT?
<400> 2
   tgggagtatc tgagcaaatt atttcttacg tgactttaga gaaaacggct acctatctga 60
   ccccaaaacg acttgaggaa actgtttcca cggtcctgct gcagagggga agcacagtcg 120
   tcaagaagag agtggggtca ggatcaaaac acatttagtg tgacttaggg aaagaaaaca 180
   ttttccctct ttgaacctct ctggatacag tcattttgcc tctacttgag gatcaactgt 240
   tcaacctca atg gcc ttt cag gac ctc ctg ggt cac gct ggt gac ctg tgg 291
   Met Ala Phe Gln Asp Leu Leu Gly His Ala Gly Asp Leu Trp
   1 5 10
   aga ttc cag atc ctt cag act gtt ttt ctc tca atc ttt gct gtt gct 339
   Arg Phe Gln Ile Leu Gln Thr Val Phe Leu Ser Ile Phe Ala Val Ala
   15 20 25 30
   aca tac ctt cat ttt atg ctg gag aac ttc act gca ttc ata cct ggc 387
   Thr Tyr Leu His Phe Met Leu Glu Asn Phe Thr Ala Phe Ile Pro Gly
   35 40 45
   cat cgc tgc tgg gtc cac atc ctg gac aat gac act gtc tct gac aat 435
   His Arg Cys Trp Val His Ile Leu Asp Asn Asp Thr Val Ser Asp Asn
   50 55 60
   gac act ggg gcc ctc agc caa gat gca ctc ttg aga atc tcc atc cca 483
   Asp Thr Gly Ala Leu Ser Gln Asp Ala Leu Leu Arg Ile Ser Ile Pro
   65 70 75
   ctg gac tca aac atg agg cca gag aag tgt cgt cgc ttt gtt cat cct 531
   Leu Asp Ser Asn Met Arg Pro Glu Lys Cys Arg Arg Phe Val His Pro
   80 85 90
   cag tgg cag ctc ctt cac ctg aat ggg acc ttc ccc aac aca agt gac 579
   Gln Trp Gln Leu Leu His Leu Asn Gly Thr Phe Pro Asn Thr Ser Asp
   95 100 105 110
   gca gac atg gag ccc tgt gtg gat ggc tgg gtg tat gac aga atc tcc 627
   Ala Asp Met Glu Pro Cys Val Asp Gly Trp Val Tyr Asp Arg Ile Ser
   115 120 125
   ttc tca tcc acc atc gtg act gag tgg gat ctg gta tgt gac tct caa 675
   Phe Ser Ser Thr Ile Val Thr Glu Trp Asp Leu Val Cys Asp Ser Gln
   130 135 140
   tca ctg act tca gtg gct aaa ttt gta ttc atg gct gga atg atg gtg 723
   Ser Leu Thr Ser Val Ala Lys Phe Val Phe Met Ala Gly Met Met Val
   145 150 155
   gga ggc atc cta ggc ggt cat tta tca gac agg ttt ggg aga agg ttc 771
   Gly Gly Ile Leu Gly Gly His Leu Ser Asp Arg Phe Gly Arg Arg Phe
   160 165 170
   gtg ctc aga tgg tgt tac ctc cag gtt gcc att gtt ggc acc tgt gca 819
   Val Leu Arg Trp Cys Tyr Leu Gln Val Ala Ile Val Gly Thr Cys Ala
   175 180 185 190
   gcc ttg gct ccc acc ttc ctc att tac tgc tca cta cgc ttc ttg tct 867
   Ala Leu Ala Pro Thr Phe Leu Ile Tyr Cys Ser Leu Arg Phe Leu Ser
   195 200 205
   ggg att gct gca atg agc ctc ata aca aat act att atg tta ata gcc 915
   Gly Ile Ala Ala Met Ser Leu Ile Thr Asn Thr Ile Met Leu Ile Ala
   210 215 220
   gag tgg gca aca cac aga ttc cag gcc atg gga att aca ttg gga atg 963
   Glu Trp Ala Thr His Arg Phe Gln Ala Met Gly Ile Thr Leu Gly Met
   225 230 235
   tgc cct tct ggt att gca ttt atg acc ctg gca ggc ctg gct ttt gcc 1011
   Cys Pro Ser Gly Ile Ala Phe Met Thr Leu Ala Gly Leu Ala Phe Ala
   240 245 250
   att cga gac tgg cat atc ctc cag ctg gtg gtg tct gta cca tac ttt 1059
   Ile Arg Asp Trp His Ile Leu Gln Leu Val Val Ser Val Pro Tyr Phe
   255 260 265 270
   gtg atc ttt ctg acc tca agt tgg ctg cta gag tct gct cgg tgg ctc 1107
   Val Ile Phe Leu Thr Ser Ser Trp Leu Leu Glu Ser Ala Arg Trp Leu
   275 280 285
   att atc aac aat aaa cca gag gaa ggc tta aag gaa ctt aga aaa gct 1155
   Ile Ile Asn Asn Lys Pro Glu Glu Gly Leu Lys Glu Leu Arg Lys Ala
   290 295 300
   gca cac agg agt gga atg aag aat gcc aga gac acc cta acc ctg gag 1203
   Ala His Arg Ser Gly Met Lys Asn Ala Arg Asp Thr Leu Thr Leu Glu
   305 310 315
   att ttg aaa tcc acc atg aaa aaa gaa ctg gag gca gca caa aaa aaa 1251
   Ile Leu Lys Ser Thr Met Lys Lys Glu Leu Glu Ala Ala Gln Lys Lys
   320 325 330
   aaa cct tct ctg tgt gaa atg ctc cac atg ccc aac ata tgt aaa agg 1299
   Lys Pro Ser Leu Cys Glu Met Leu His Met Pro Asn Ile Cys Lys Arg
   335 340 345 350
   atc tcc ctc ctg tcc ttt acg aga ttt gca aac ttt atg gcc tat ttt 1347
   Ile Ser Leu Leu Ser Phe Thr Arg Phe Ala Asn Phe Met Ala Tyr Phe
   355 360 365
   ggc ctt aat ctc cat gtc cag cat ctg ggg aac aat gtt ttc ctg ttg 1395
   Gly Leu Asn Leu His Val Gln His Leu Gly Asn Asn Val Phe Leu Leu
   370 375 380
   cag act ctc ttt ggt gca gtc atc ctc ctg gcc aac tgt gtt gca cct 1443
   Gln Thr Leu Phe Gly Ala Val Ile Leu Leu Ala Asn Cys Val Ala Pro
   385 390 395
   tgg gca ctg aaa tac atg aac cgt cga gca agc cag atg ctt ctc atg 1491
   Trp Ala Leu Lys Tyr Met Asn Arg Arg Ala Ser Gln Met Leu Leu Met
   400 405 410
   ttc cta ctg gca atc tgc ctt ctg gcc atc ata ttt gtg cca caa gaa 1539
   Phe Leu Leu Ala Ile Cys Leu Leu Ala Ile Ile Phe Val Pro Gln Glu
   415 420 425 430
   atg cag acg ctg cgt gag gtt ttg gca aca ctg ggc tta gga gcg tct 1587
   Met Gln Thr Leu Arg Glu Val Leu Ala Thr Leu Gly Leu Gly Ala Ser
   435 440 445
   gct ctt gcc aat acc ctt gct ttt gcc cat gga aat gaa gta att ccc 1635
   Ala Leu Ala Asn Thr Leu Ala Phe Ala His Gly Asn Glu Val Ile Pro
   450 455 460
   acc ata atc agg gca aga gct atg ggg atc aat gca acc ttt gct aat 1683
   Thr Ile Ile Arg Ala Arg Ala Met Gly Ile Asn Ala Thr Phe Ala Asn
   465 470 475
   ata gca gga gcc ctg gct ccc ctc atg atg atc cta agt gtg tat tct 1731
   Ile Ala Gly Ala Leu Ala Pro Leu Met Met Ile Leu Ser Val Tyr Ser
   480 485 490
   cca ccc ctg ccc tgg atc atc tat gga gtc ttc ccc ttc atc tct ggc 1779
   Pro Pro Leu Pro Trp Ile Ile Tyr Gly Val Phe Pro Phe Ile Ser Gly
   495 500 505 510
   ttt gct ttc ctc ctc ctt cct gaa acc agg aac aag cct ctg ttt gac 1827
   Phe Ala Phe Leu Leu Leu Pro Glu Thr Arg Asn Lys Pro Leu Phe Asp
   515 520 525
   acc atc cag gat gag aaa aat gag aga aaa gac ccc aga gaa cca aag 1875
   Thr Ile Gln Asp Glu Lys Asn Glu Arg Lys Asp Pro Arg Glu Pro Lys
   530 535 540
   caa gag gat ccg aga gtg gaa gtg acg cag ttt taa ggaattccag 1921
   Gln Glu Asp Pro Arg Val Glu Val Thr Gln Phe
   545 550
   gagctgactg ccgatcaatg agccagatga agggaacaat caggactatt cctagacact 1981
   agcaaaatct agaaaataaa taacaaggct gggtgcggtg gctcacgcct gtaatcccag 2041
   ccccttggga ggctgaggcg ggcagatcat gaggtcagaa gataaagacc cccctggcca 2101
   acatggtgaa accctgtctc tactaaaaca aatacaaaac ttcgctgggc acagtggcac 2161
   aggcctttaa ttccagctac ttgggaggct gaggcaggag aattacttga acccaggagg 2221
   tggaaattgc aatgagccaa gattgggccc ctgcattcca gcctggtgac agagcgagac 2281
   tgtctcaaaa aaaaaaaaaa aaaaaaagaa ggaaagaaag aaagaaaaaa aaaaaaaaaa 2341
   a 2342
<210> 3
   <211> 12
   <212> PRT
   <213> Artificial
<220>
   <221> misc_feature
   <223> synthetic peptide corresponding to partial sequence 542-552 of OA T7
<400> 3
   Cys Lys Gln Glu Asp Pro Arg Val Glu Val Thr Gln
   1 5 10